# EUROPÄISCHE PATENTSCHRIFT

(11) **EP 2 149 388 B1**
(45) Veröffentlichungstag und Bekanntmachung des Hinweises auf die Patenterteilung: **02.02.2011**
(21) Anmeldenummer: 08013836.5
(22) Anmeldetag: 01.08.2008
(51) Int. Cl.: A61M 37/00

(54) **Handgerät zum lokalen Aufstechen einer menschlichen oder tierischen Haut, Antriebsmodul, Nadelmodul sowie Verfahren zum Ankoppeln**
Handheld device for local punctuation of human or animal skin, drive module, needle module and method for coupling
Appareil manuel destiné au perçage local d'une peau humaine ou animale, module d'entraînement, module d'aiguille et procédé d'accouplement

(43) Veröffentlichungstag der Anmeldung: 03.02.2010
(73) Patentinhaber: MT Derm GmbH, 14167 Berlin (DE); Technische Universität Berlin, 10623 Berlin (DE)
(72) Erfinder: Oginski, Stefan, 10369 Berlin (DE); Knothe, Kornelius, 13089 Berlin (DE)
(74) Vertreter: Bittner, Thomas L.

(56) Entgegenhaltungen:
- EP-A- 1 882 491
- WO-A-2008/018781
- DE-A1- 19 836 376
- DE-A1- 19 945 334

## Beschreibung

Die Erfindung betrifft ein Handgerät zum lokalen Aufstechen einer menschlichen oder tierischen Haut, insbesondere zum Einbringen eines Wirkstoffs, zum Tätowieren oder zum Aufbringen von permanentem Make-up, sowie ein Antriebsmodul und ein Nadelmodul für das Handgerät und ein Verfahren zum Ankoppeln des Nadelmoduls an das Antriebsmodul.

### Hintergrund der Erfindung

Derartige Handgeräte werden benutzt, um mittels einer repetierenden Aus- und Einfahrbewegung eines Nadelelementes, welches ein oder mehrere Nadeln umfassen kann, eine menschliche oder eine tierische Haut lokal aufzustechen. Im Bereich der aufgestochenen Haut können dann beliebige Stoffe in die Haut eingetragen werden, insbesondere ein Farbstoff, ein kosmetischer Stoff oder ein pharmazeutischer Wirkstoff. Üblicherweise wird die repetierende Ausund Einfahrbewegung hochfrequent ausgeführt.

Bekannte Handgeräte (siehe zum Beispiel DE 198 36 376 oder WO 2008/018781) zum lokalen Aufstechen einer Haut verfügen über ein Antriebsmodul, welches seinerseits Antriebsmittel aufweist, die die repetierende Antriebsbewegung bereitstellen. Die repetierende Antriebsbewegung kann auf das Nadelelement übertragen werden, indem an eine Nadelanschlusseinrichtung eine Nadeleinrichtung mit dem Nadelelement angeschlossen wird.

### Zusammenfassung der Erfindung

Aufgabe der Erfindung ist es, neue Technologien für ein Handgerät zum lokalen Aufstechen einer menschlichen oder tierischen Haut anzugeben, die ein die Funktion des Handgerätes sicherstellendes und bedienerfreundliches Koppeln / Entkoppeln zwischen einem Antriebsmodul und einem Nadelmodul des Handgerätes ermöglichen.

Diese Aufgabe wird erfindungsgemäß durch ein Handgerät nach dem unabhängigen Anspruch 1 gelöst. Weiterhin sind ein Verfahren zum Ankoppeln eines Nadelmoduls an ein Antriebsmodul bei dem Handgerät nach dem unabhängigen Anspruch 14 sowie ein Antriebsmodul für das Handgerät nach dem unabhängigen Anspruch 15 und ein Nadelmodul für das Handgerät nach dem unabhängigen Anspruch 16 vorgesehen. Vorteilhafte Ausgestaltungen der Erfindung sind Gegenstand von abhängigen Unteransprüchen.

Die Erfindung umfasst den Gedanken eines Handgerätes zum lokalen Aufstechen einer menschlichen oder tierischen Haut mit einem Antriebsmodul, welches konfiguriert ist, eine repetierende Antriebsbewegung bereitzustellen, und einem Nadelmodul, welches eine Nadeleinrichtung aufweist und konfiguriert ist, an das Antriebsmodul zu koppeln, derart, dass die repetierende Antriebsbewegung zum Ein- / Ausfahren der Nadeleinrichtung auf diese einkoppelbar ist, wobei an dem Antriebsmodul ein verlagerbares, an eine Nadelanschlusseinrichtung funktionell gekoppeltes Betätigungselement und an dem Nadelmodul ein dem Betätigungselement zugeordnetes Funktionselement gebildet sind, die konfiguriert sind, beim Ankoppeln des Nadelmoduls an das Antriebsmodul mit dem zugeordneten Funktionselement das Betätigungselement aus einer ersten in eine zweite Verlagerungsstellung zu verlagern, wodurch die Nadelanschlusseinrichtung in eine Kopplungsstellung gebracht wird, in welcher die Nadeleinrichtung zum Betrieb an die Nadelanschlusseinrichtung koppelbar ist.

Nach einem weiteren Aspekt der Erfindung ist ein Verfahren zum Ankoppeln eines Nadelmoduls an ein Antriebsmodul bei dem Handgerät geschaffen, wobei das Verfahren die folgenden Schritte umfasst: Bereitstellen eines Antriebsmoduls, welches konfiguriert ist, eine repetierende Antriebsbewegung bereitzustellen, Bereitstellen eines Nadelmoduls, welches eine Nadeleinrichtung aufweist und konfiguriert ist, an das Antriebsmodul zu koppeln, derart, dass die repetierende Antriebsbewegung zum Ein- / Ausfahren der Nadeleinrichtung auf diese einkoppelbar ist, und Ankoppeln des Nadelmoduls an das Antriebsmodul, indem ein verlagerbares, an eine Nadelanschlusseinrichtung funktionell gekoppeltes Betätigungselement des Antriebsmoduls mittels eines dem Betätigungselement zugeordneten Funktionselement des Nadelmoduls aus einer ersten in eine zweite Verlagerungsstellung verlagert wird, wodurch die Nadelanschlusseinrichtung in eine Kopplungsstellung gebracht wird, und die Nadeleinrichtung dann zum Betrieb an die Nadelanschlusseinrichtung gekoppelt wird.

Weiterhin ist ein Antriebsmodul für ein Handgerät geschaffen mit Antriebsmitteln, welche konfiguriert sind, eine repetierende Antriebsbewegung bereitzustellen, einer Nadelanschlusseinrichtung und einem verlagerbaren Betätigungselement, welches an die Nadelanschlusseinrichtung funktionell gekoppelt und konfiguriert ist, beim Ankoppeln eines Nadelmoduls mit einem hieran gebildeten und dem Betätigungselement zugeordneten Funktionselement das Betätigungselement aus einer ersten in eine zweite Verlagerungsstellung zu verlagern, wodurch die Nadelanschlusseinrichtung in eine Kopplungsstellung gebracht wird, in welcher eine von dem Nadelmodul umfassten Nadeleinrichtung zum Betrieb an die Nadelanschlusseinrichtung koppelbar ist.

Schließlich ist ein Nadelmodul für ein Handgerät geschaffen mit einer Nadeleinrichtung und einem Funktionselement, welches einem verlagerbaren Betätigungselement eines ankoppelbaren Antriebsmoduls zugeordnet und konfiguriert ist, beim Ankoppeln an das Antriebsmodul das Betätigungselement aus einer ersten in eine zweite Verlagerungsstellung zu verlagern, wodurch eine von dem Antriebsmodul umfasste Nadelanschlusseinrichtung in eine Kopplungsstellung gebracht wird, in welcher die Nadeleinrichtung zum Betrieb an die Nadelanschlusseinrichtung koppelbar ist.

Mit Erfindung ist auf einfache aber zuverlässige Art und Weise sichergestellt, dass die Nadelanschlusseinrichtung, welche ihrerseits in dem Antriebsmodul die Kopplungsstelle für die Nadeleinrichtung des Nadelmoduls bildet, beim Ankoppeln zuverlässig in die Kopplungsstellung gebracht wird, in welcher dann die Nadeleinrichtung zum Betrieb angekoppelt werden kann. Das Betätigungselement, welches funktionell an die Nadelanschlusseinrichtung gekoppelt ist, und das dem Betätigungselement zugeordnete Funktionselement an dem Nadelmodul wirken als Ankopplungsmechanismus zusammen, um im Prozess der Ankopplung die Nadelanschlusseinrichtung in die Kopplungsstellung zu bringen, welche dann den Anschluss der Nadeleinrichtung an die Nadelanschlusseinrichtung ermöglicht.

Das Funktionselement an dem Nadelmodul kann beispielsweise als ein Anschlagelement ausgeführt sein, welches beim Ankoppeln des Nadelmoduls an das Antriebsmodul mit dem Betätigungselement in Kontakt gebracht wird und dieses in die zweite Verlagerungsstellung verlagert. Ein solcher Anschlagmechanismus ist mechanisch besonders einfach umsetzbar. Alternativ oder ergänzend können jedoch auch andere Wirkmechanismen für das Zusammenwirken von zugeordnetem Funktionselement und Betätigungselement eingesetzt werden, beispielsweise ein magnetischer Wirkmechanismus, welcher die Verlagerung des Betätigungselementes aus der ersten in die zweite Verlagerungsstellung veranlasst.

Eine bevorzugte Weiterbildung der Erfindung sieht vor, dass das Betätigungselement konfiguriert ist, sich beim Abkoppeln des Nadelmoduls von dem Antriebsmodul selbsttätig aus der zweiten in die erste Verlagerungsstellung zu verlagern, wodurch die Nadelanschlusseinrichtung in eine Abkopplungsstellung gebracht wird, in welcher die Nadeleinrichtung von der Nadelanschlusseinrichtung abkoppelbar ist. Beispielweise kann die selbsttätige Rückverlagerung des Betätigungselementes erreicht werden, indem mittels eines Felderbauteils eine Federspannung bereitgestellt ist. Gegen die Federkraft wird das Betätigungselement aus der ersten in die zweite Verlagerungsstellung gebracht. Beim Lösen des Nadelmoduls von dem Antriebsmodul sorgt die Federkraft in dieser Ausgestaltung dann dafür, dass das Betätigungselement, welches seinerseits funktionell an die Nadelanschlusseinrichtung gekoppelt ist, in die Ausgangsstellung zurückgeht.

Bevorzugt sieht eine Fortbildung der Erfindung vor, dass das Antriebsmodul und das Nadelmodul konfiguriert sind, eine formschlüssige Kopplungsverbindung einzugehen. Mit einer formschlüssigen Verbindung kann einerseits zum Beispiel ein fester Sitz des Nadelmoduls an dem Antriebsmodul unterstützt werden. Darüber hinaus kann in einer möglichen Ausgestaltung so sicherstellt werden, dass eine funktionssichernde korrekte Anordnung von Antriebsmodul und Nadelmodul relativ zueinander gewährleistet ist, so dass das Nadelmodul funktionsgerecht an dem Antriebsmodul montiert ist. Unanhängig von der formschlüssigen Kopplungsverbindung oder in Kombination hiermit kann ein Führungsmechanismus vorgesehen sein, welcher das Nadelmodul beim Ankoppeln an das Antriebsmodul führt, beispielsweise mittels eines Vorsprungs an dem Antriebsmodul, welcher in eine Führungsvertiefung an dem Nadelmodul eingreift, die zum Beispiel auf einer inneren Oberfläche eines Gehäuse des Nadelmoduls gebildet ist.

Bei einer vorteilhaften Ausgestaltung der Erfindung kann vorgesehen sein, dass das Nadelmodul einen proximalen Abschnitt, an dem das zugeordnete Funktionselement gebildet ist und welcher konfiguriert ist, an das Antriebsmodul zu koppeln, und einen hiermit verbundenen distalen Abschnitt aufweist, in welchem eine Nadelöffnung gebildet ist, durch welche hindurch ein Nadelelement der Nadeleinrichtung ein- und ausgefahren werden kann. Bevorzugt ist das Nadelmodul mit proximalem und distalem Abschnitt einteilig ausgeführt. In einer Ausgestaltung ist das Gehäuse des Nadelmoduls im Bereich des proximalen Abschnitts elastisch verformbar ausgestaltet, beispielsweise als ein Kunststoffgehäuse, welches es dem Benutzer ermöglicht, den proximalen Abschnitt mittels Fingerkraft zu verformen, so dass ein leichteres An- / Abkoppeln des Nadelmoduls oder das Ausführen des Kopplungsprozesses überhaupt ermöglicht sind. Beispielweise kann in einer Ausführung mittels des elastischen Verformens des proximalen Abschnitts mittels Fingerkraft eine Rastverbindung zwischen dem Nadelmodul und dem Antriebsmodul gelöst werden.

Eine bevorzugte Weiterbildung der Erfindung sieht vor, dass der proximale Abschnitt und der distale Abschnitt lösbar miteinander verbunden sind. Mit dieser Ausführung kann beispielsweise eine Ausgestaltung des Handgerätes realisiert werden, bei der zunächst der proximale Abschnitt an das Antriebsmodul gekoppelt wird, wobei hierbei das Betätigungselement und somit die Nadelanschlusseinrichtung betätigt werden. Anschließend kann in dieser Ausgestaltung dann der distale Abschnitt mit der Nadeleinrichtung angeschlossen werden, beispielsweise mittels Aufstecken oder Aufschrauben. Hierbei wird dann auch die Nadeleinrichtung an die sich in der Kopplungsstellung befindliche Nadelanschlusseinrichtung angekoppelt. Eine weitere Ausgestaltung sieht vor, dass der proximale Abschnitt des Nadelmoduls an dem Antriebsmodul zwei Rastpositionen einnehmen kann, derart dass das zugehörige Betätigungselement des Antriebsmoduls die erste Verlagerungsstellung einnimmt, wenn der proximale Abschnitt die erste Rastposition einnimmt und dass das zugehörige Betätigungselement des Antriebsmoduls die zweite Verlagerungsstellung einnimmt, wenn der proximale Abschnitt die zweite Rastposition einnimmt. Hierdurch wird die Nadelanschlusseinrichtung in die Kopplungs-/ Abkopplungsstellung gebracht, wenn der proximale Abschnitt des Nadelmoduls sich in der zweiten / ersten Raststellung befindet. Dadurch kann der distale Abschnitt des Nadelmoduls von dem proximalen Abschnitt abgezogen oder hiermit verbunden werden, wenn der proximale Abschnitt die erste Rastposition einnimmt. Somit kann der distale Abschnitt gewechselt werden, während der proximale Abschnitt des Nadelmoduls mit dem Antriebsmodul verbunden bleibt.

Eine vorteilhafte Ausfiihrungsform der Erfindung sieht vor, dass in einer angekoppelten Stellung des Nadelmoduls an dem Antriebsmodul der proximale Abschnitt an dem Antriebsmodul fixiert ist. Die Fixierung kann beispielsweise mit Hilfe eines Rast- oder eines Anschlagmechanismus implementiert sein.

Bei einer zweckmäßigen Ausgestaltung der Erfindung kann vorgesehen sein, dass beim Ankoppeln des Nadelmoduls an das Antriebsmodul der proximale Abschnitt an einem Gehäuse des Antriebsmoduls geführt ist. Die Führung erfolgt beispielsweise mittels einer Kombination eines Vorsprungs und einer den Vorsprung aufnehmenden Führungsnut.

Eine vorteilhafte Ausführungsform der Erfindung sieht vor, dass das Betätigungselement konfiguriert ist, sich zwischen der ersten und der zweiten Verlagerungsstellung in Richtung einer beim Ankoppeln des Nadelmoduls an das Antriebsmodul von dem Nadelmodul ausgeführten Ankopplungsbewegung zu verlagern. In einer bevorzugten Ausgestaltung ist die Ankopplungsbewegung eine geradlinige Bewegung.

Bevorzugt sieht eine Fortbildung der Erfindung vor, dass das Betätigungselement in einer Führung an dem Antriebsmodul gelagert ist, welche konfiguriert ist, das Betätigungselement beim Übergang zwischen der ersten und der zweiten Verlagerungsstellung zu führen. Die Führung des Betätigungselementes erfolgt beispielsweise mit Hilfe einer an dem Gehäuse des Antriebsmodul gebildeten Führung, zum Beispiel mit Hilfe ineinander verschiebbar gelagerter Bauteile. In einer Ausführungsform ist das Betätigungselement als eine zylindrische Hülle gebildet, die relativ zu Gehäuseabschnitten des Antriebsmoduls verlagerbar ist, beispielsweise mittels eines Gleitbewegung. Bei dieser oder anderen Ausgestaltungen kann das Betätigungselement als ein Gehäuseelement des Antriebsmoduls ausgeführt sein.

Eine bevorzugte Weiterbildung der Erfindung sieht vor, dass die Nadelanschlusseinrichtung mit einem mechanischen Anschluss-Mechanismus für die Nadeleinrichtung gebildet ist. Der Anschluss-Mechanismus kann in einer Ausführungsform beispielsweise ein Federkraft-Anschluss-Mechanismus sein, bei dem die Nadeleinrichtung in der Nadelanschlusseinrichtung mittels einer Federkraftklemmung gehalten wird. Aber auch andere Klemmmechanismen können genutzt werden. Darüber hinaus kann beispielsweise ergänzend oder alternativ eine Ankopplung unter Einbeziehung eines Magnetmechanismus vorgesehen sein. Ein solcher Magnetmechanismus kann mittels Dauermagneten oder Elektromagneten zur Verfügung gestellt werden.

In einer Ausgestaltung weist die Nadelanschlusseinrichtung einander zugeordnete und relativ zueinander verlagerbare Klemm- oder Befestigungselemente auf, die beim Betätigen des Betätigungselementes relativ zueinander bewegt werden, so dass unterschiedliche Funktionsstellungen eingenommen werden. Die Relativbewegung der Klemm- oder Befestigungselemente kann mit Hilfe von Spreizelementen realisiert sein, die zum Beispiel in Form von bewegbaren oder feststehenden Stößeln oder Vorsprüngen hergestellt sind. Bevorzugt wird der Übergang zwischen den Funktionsstellungen der Klemmelemente mittels einer Bewegung in Richtung der Ankoppelbewegung erreicht. Unabhängig von der konkreten Ausgestaltung der Nadelanschlusseinrichtung kann diese in einer Ausführungsform konfiguriert sein, dass eine formschlüssige Verbindung zwischen der Nadeleinrichtung und der Nadelanschlusseinrichtung beim Ankoppeln gebildet wird.

Eine vorteilhafte Ausführungsform der Erfindung sieht vor, dass die Nadelanschlusseinrichtung konfiguriert ist, die Nadeleinrichtung starr anzukoppeln. Dies bedeutet in einer möglichen Ausfiihrungsform insbesondere, dass sowohl die Schubkraft zum Ausfahren der Nadeleinrichtung als auch die Rückholkraft zum Einfahren der Nadeleinrichtung über die Nadelanschlusseinrichtung auf die Nadeleinrichtung und hiermit auf das Nadelelement direkt gekoppelt werden, zum Beispiels mittels einer form- oder kraftschlüssigen Verbindung. Hierdurch werden auch ungewünscht Klappergeräusche beim Betrieb des Handgerätes vermieden. Vorteilhaft ist in diesem Zusammenhang zum Beispiel die formschlüssige Kopplung zwischen Nadelanschlusseinrichtung und Nadeleinrichtung, wobei letztere hierzu einen entsprechend konfigurierten Nadelschaft aufweisen kann. Eine formschlüssige Verbindung kann beispielsweise mittels Vertiefungen an dem Nadelschaft und hierin formschlüssig eingreifenden Funktionselementen an der Nadelanschlusseinrichtung gebildet sein. Ergänzend oder alternativ kann eine kraft- oder reibschlüssige Verbindung gebildet sein, die beispielsweise mit Federelementen die radial auf die Nadelanschlusseinrichtung drücken, realisiert werden kann. Durch die wirkende Klemmkraft und die vorhandene Reibung zwischen Federelement und Nadeleinrichtung kann die Kupplung die zum Tätowieren nötigen Kräfte in axialer Richtung übertragen.

Eine bevorzugte Weiterbildung der Erfindung sieht vor, dass die Nadeleinrichtung in dem Nadelmodul in einem die Nadeleinrichtung beim Ankoppeln des Nadelmoduls an das Antriebsmodul in einer Ausgangsstellung haltenden Führungselement gelagert ist. Das Führungselement, welches zum Beispiel aus einem elastischen Material ist, ist beispielsweise mittels einer das Nadelelement umgreifenden Membran gebildet. Das Führungselement hat die Aufgabe, die Nadeleinrichtung im Modul derart zu positionieren, dass ein zuverlässiges Ankoppeln der Nadeleinrichtung an die Nadelanschlusseinrichtung ermöglicht wird. Die für die Positionierung erforderlichen Stellkräfte sind allerdings außerordentlich gering und liegen nur im Bereich der Gewichtskraft der Nadeleinrichtung. Somit braucht das Führungselement nur mit entsprechend geringer Steifigkeit ausgelegt werden. Es hat auf Grund der geringen Stellkräfte praktisch keinen Einfluss auf die repierende Bewegung während des Tätowiervorgangs und erlaubt beim Betrieb des Handgerätes eine freie Bewegung des Nadelelements, so dass die Aus- und Einfahrbewegung des Nadelelementes im Wesentlichen frei von Rückwirkungen hierauf durch das Führungselement sind. In einer Ausgestaltung weist die Membran zu diesem Zweck einen domförmigen Querschnitt auf, bei dem sich die Membranwandung zur Mitte hin wieder absenkt. Weiterhin ist das Führungselement hierbei zugleich eine Dichtung gegenüber der Austrittsöffnung in dem Nadelmodul. Hierdurch wird bei der Nutzung des Handgeräts ein Übertreten des in die Haut einzubringenden Stoffes oder von aus der Haut austretendem Blut von dem Bereich hinter der Austrittsöffnung in andere Abschnitte des Nadelmoduls oder des Antriebsmoduls verhindert. Als weitere Ausführungsform kann anstelle einer federnden Membran geringer Steifigkeit auch eine Kombination aus einer Gleitdichtung, durch welche hindurch das Nadelelement aus- und eingefahren wird, und einer schwachen Feder, die die Nadeleinrichtung für den Kopplungsvorgang positioniert, verwendet werden.

Bevorzugt sieht eine Fortbildung der Erfindung vor, dass das Nadelmodul als ein Einwegmodul ausgeführt ist. Ist das Nadelmodul mit voneinander trenn- oder lösbaren Abschnitten gebildet, kann vorgesehen sein, dass nur der distale Abschnitt mit dem Nadelelement als Einwegartikel ausgeführt ist.

Das Verfahren zum Ankoppeln des Nadelmoduls an das Antriebsmodul bei dem Handgerät sowie das Nadelmodul und das Antriebsmodul können eine oder mehrere der vorangehend erläuterter Ausgestaltungen entsprechend ausgeführt sein.

### Beschreibung bevorzugter Ausführtmgsbespeispiele der Erfindung

Die Erfindung wird im Folgenden anhand von bevorzugten Ausführungsbespielen unter Bezugnahme auf Figuren einer Zeichnung näher erläutert. Hier zeigen:
- Fig. 1: eine perspektivische Darstellung eines Handgeräts, bei dem ein Nadelmodul an ein Antriebsmodul gekoppelt ist,
- Fig. 2: eine perspektivische Darstellung des Handgeräts aus Fig. 1, wobei das Nadelmodul von dem Antriebsmodul entkoppelt ist,
- Fig. 3: eine perspektivische Darstellung des Handgeräts nach Fig. 2 im Aufriss im entkop- pelten Zustand,
- Fig. 4: eine perspektivische Darstellung eines Abschnitts des Handgeräts nach Fig. 1 im Aufriss im entkoppelten Zustand,
- Fig. 5: mehrere Darstellungen einer Nadelanschlusseinrichtung des Handgeräts nach Fig. 1,
- Fig. 6: eine perspektivische Darstellung eines Abschnitts des Handgeräts nach Fig. 1 im Aufriss im gekoppelten Zustand,
- Fig. 7: eine Querschnittsdarstellung des Abschnitts des Handgeräts aus Fig. 6,
- Fig. 8: eine vergrößerte Detaildarstellung beim Ankoppeln einer Nadeleinrichtung,
- Fig. 9: eine vergrößerte Darstellung der Nadelanschlusseinrichtung mit angekoppelter Na- deleinrichtung,
- Fig. 10: eine perspektivische Darstellung mit Nadelanschlusseinrichtung und Betätigungs- element nach einer weiteren Ausfiihrungsform,
- Fig. 11: eine perspektivische Darstellung der Anordnung aus Fig. 10 im gekoppelten Zu- stand,
- Fig. 12: eine perspektivische Darstellung der Anordnung aus Fig. 10, wobei die Nadelan- schlusseinrichtung in einer Abkopplungsstellung ist,
- Fig. 13: eine perspektivische Darstellung eines Abschnitts des Antriebsmoduls,
- Fig. 14: eine perspektivische Darstellung eines Abschnittes eines Handgeräts, bei dem ein Nadelmodul mehrteilig ausgeführt ist, und
- Fig. 15: eine weitere perspektivische Darstellung des Abschnittes des Handgeräts nach Fig. 15.

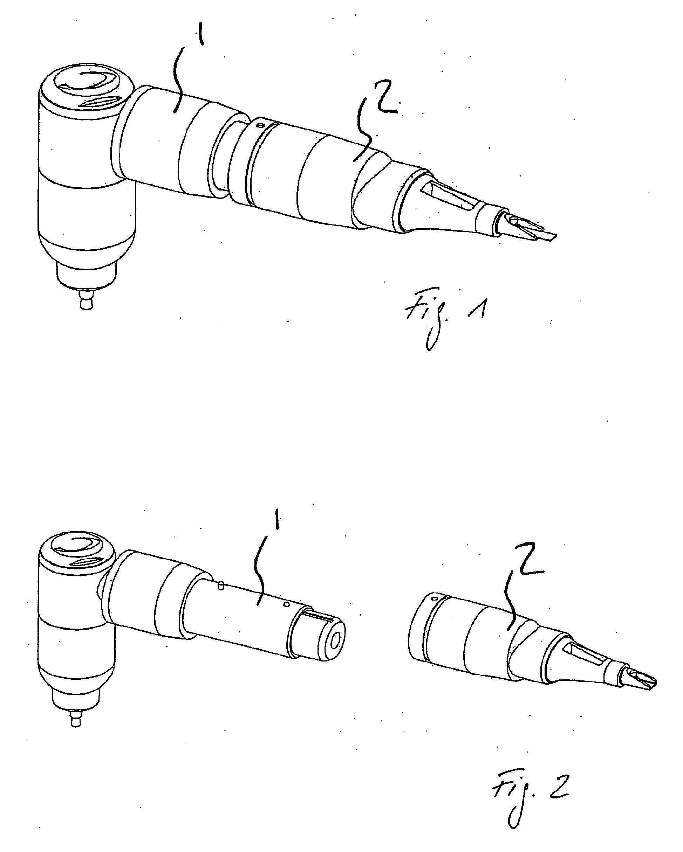

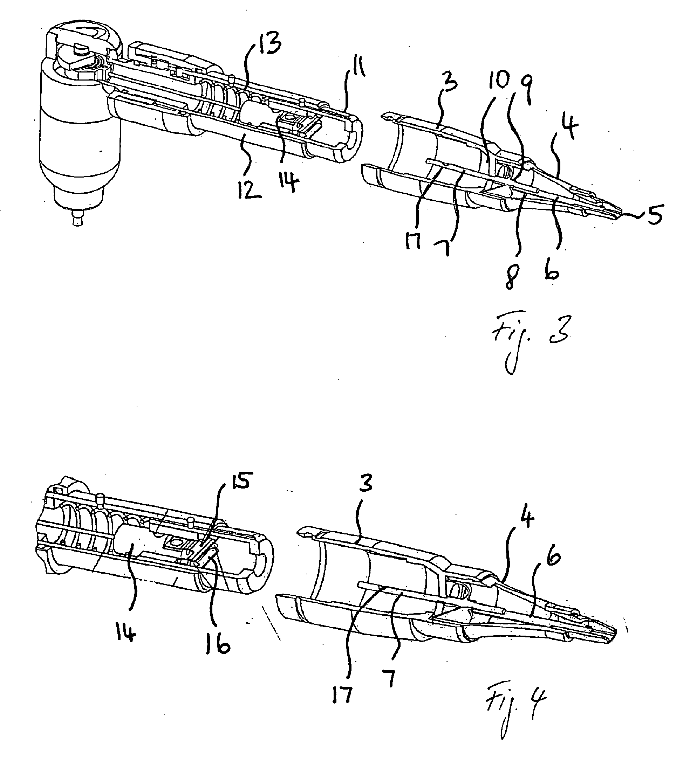

Fig. 1 zeigt eine perspektivische Darstellung eines Handgeräts zum lokalen Aufstechen einer menschlichen oder einer tierischen Haut, bei dem ein Antriebsmodul 1 und ein Nadelmodul 2 gekoppelt sind. Fg. 2 zeigt eine perspektivische Darstellung des Handgeräts aus Fig. 1, wobei das Nadelmodul 2 von dem Antriebsmodul 1 entkoppelt ist. Fig. 3 zeigt eine perspektivische Darstellung des Handgeräts nach Fig. 2 im Aufriss im entkoppelten Zustand.

Das Nadelmodul 2 weist einen proximalen Abschnitt 3, welcher unabhängig von der konkreten Ausführungsform auch als Kopplungsabschnitt bezeichnet werden kann, sowie einen distalen Abschnitt 4 auf, welcher unabhängig von der konkreten Ausführungsform auch als Nadelaufnahmeabschnitt bezeichnet werden kann und in welchem eine Nadelöffnung 5 gebildet ist, durch die hindurch ein Nadelelement 6 aus- und eingefahren werden kann. Das Nadelelement 6, welches mit einer oder mehreren Nadeln gebildet sein kann, ist seinerseits in einen Nadelschaft 7 aufgenommen, welcher mit dem Nadelelement 6 eine Nadeleinrichtung 8 bildet. Die Nadeleinrichtung 8 wird mittels einer Membran 9 geführt. Die Membran 9 ist aus einem elastischen Material und hält die Nadeleinrichtung 8 im in Fig. 3 gezeigten entkoppelten Zustand in einer Ausgangsstellung. Im Betrieb, das heißt wenn Antriebsmodul 1 und Nadelmodul 2 verbunden sind und von dem Antriebsmodul 1 eine repetierende Antriebsbewegung zur Verfügung gestellt wird, wird die an der Nadeleinrichtung 8 befestigte Membran 9 gedehnt, ohne dass diese der Antriebsbewegung im Betrieb wirksame Kräfte entgegensetzt.

An dem Nadelmodul 2 ist am proximalen Abschnitt 3 ein als Anschlagelement ausgeführtes Funktionselement 10 gebildet. Das Funktionselement 10 wirkt beim Ankoppeln des Nadelmoduls 2 an das Antriebsmodul 1 mit einem Betätigungselement 11 zusammen, was unten in den Fig. 4, 6 und 7 deutlicher wird.

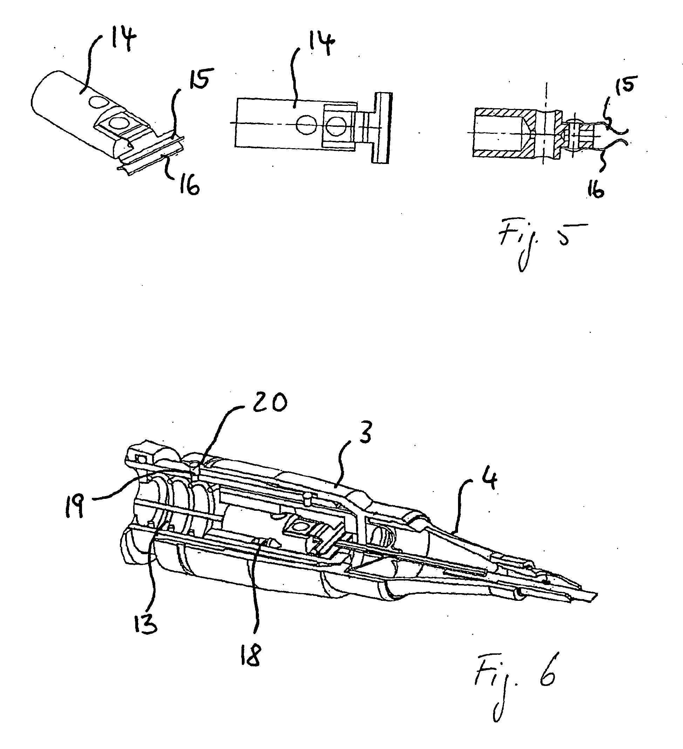

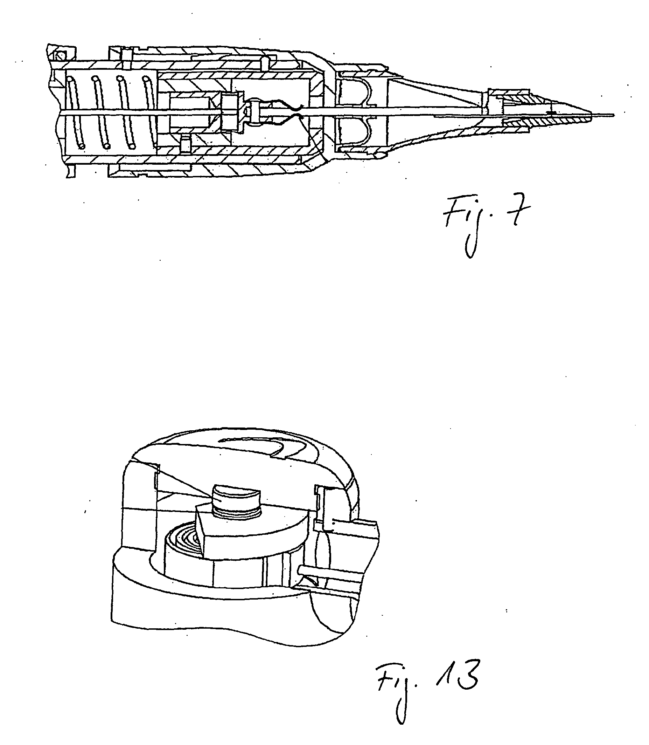

Das Betätigungselement 11 ist bei der dargestellten Ausführungsform als eine zylindrische Hülse gebildet, die in einem Gehäuseabschnitt 12 verschiebbar gelagert ist. Die Verlagerung des Betätigungselementes 11 aus einer ersten Verlagerungsstellung, die in Fig. 3 gezeigt ist und die einer ausgefahrenen Stellung entspricht, in eine eingefahrene Stellung (vgl. Fig. 6 und 7) erfolgt gegen die Vorspannung eines Federelementes 13 beim Ankoppeln des Nadelmoduls 2 an das Antriebsmodul 1.

Das Betätigungselement 11 ist funktionell an eine in dem Gehäuseabschnitt 12 angeordnete Nadelanschlusseinrichtung 14 gekoppelt, derart, dass beim Einschieben des Betätigungselementes 11 die Nadelanschlusseinrichtung 14 betätigt wird, so dass die Nadelanschlusseinrichtung 14 in eine Kopplungsstellung geht, die in Fig. 5 für die Nadelanschlusseinrichtung 14 gezeigt ist. In der Kopplungsstellung sind gemäß Fig. 5 als Klemm- oder Befestigungselemente ausgeführte Funktionselemente 15, 16 gegenüberliegend in einem Abstand angeordnet, welcher es dem Nadelschaft 7 ermöglicht, hieran zu koppeln, derart, dass die Funktionselemente 15, 16 in eine Vertiefung 17 am Nadelschaft 7 formschlüssig eingreifen, was insbesondere in Fig. 7 zu sehen ist.

Die Funktionselemente 15, 16 der Nadelanschlusseinrichtung 14 gelangen beim Einschieben des Betätigungselementes 11 im Prozess der Ankopplung des Nadelmoduls 2 in die in Fig. 5 gezeigte Kopplungsstellung, indem keilförmige Stößel 18, die fest mit dem Betätigungselement 11 verbunden sind, zurückgefahren werden, was insbesondere in Fig. 6 zu sehen ist. Der proximale Abschnitt 3 des Nadelmoduls 2 wird hierbei soweit auf den Gehäuseabschnitt 12 geschoben, dass gemäß Fig. 6 ein Haltestift 19 in eine Bohrung 20 in dem proximalen Abschnitt 3 des Nadelmoduls eingreift, wodurch das Nadelmodul 2 an dem Antriebsmodul 1 fixiert ist. Zum Lösen aus dieser fixierten Stellung kann der proximale Abschnitt 3 mittels Fingerkraft von dem Benutzer elastisch verformt werden, so dass sich die Bohrung 20 von dem Haltestift 19 löst, was das Abnehmen des Nadelmoduls 2 ermöglicht. Bei dieser Entkopplung geht dann das Betätigungselement, getrieben von dem Federelement 13, wieder in die in Fig. 3 gezeigte Stellung zurück. In Fig. 3 ist auch erkennbar, dass dann die keilförmigen Stößel 18 die Funktionselemente 15, 16 der Nadelanschlusseinrichtung 14 auseinanderdrücken, so dass die Nadeleinrichtung 8 aus der Nadelanschlusseinrichtung 14 gelöst werden kann.

Entgegen der Darstellung in Fig. 6 ist die Membran 9 in dem dort gezeigten gekoppelten Zustand verformt und gedehnt, da sie fest auf dem Nadelschaft 7 sitzt.

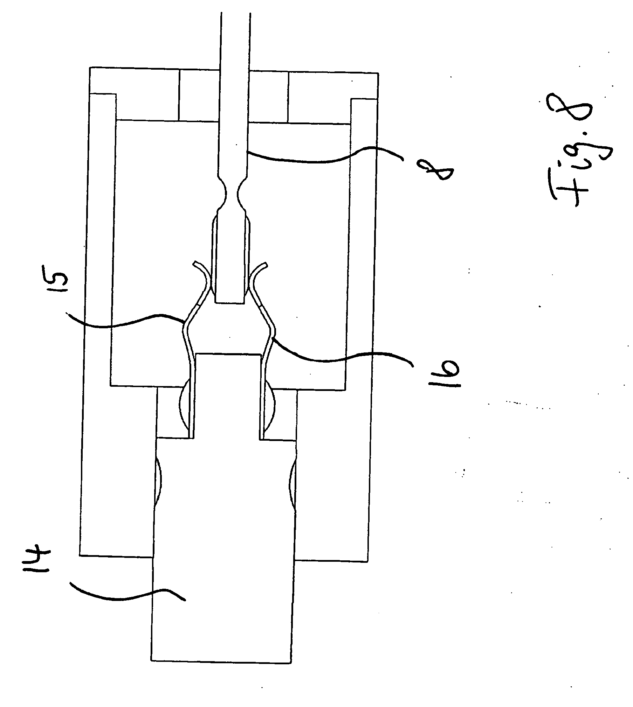

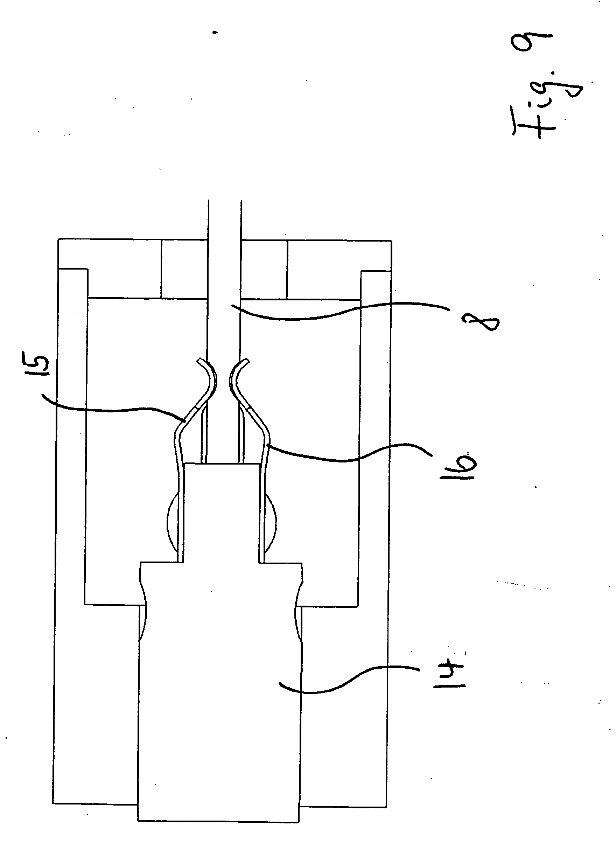

Fig. 8 zeigt eine vergrößerte Detaildarstellung beim Ankoppeln der Nadeleinrichtung 8 an die Nadelanschlusseinrichtung 14. Fig. 9 zeigt eine vergrößerte Darstellung der Nadelanschlusseinrichtung 14 mit angekoppelter Nadeleinrichtung 8.

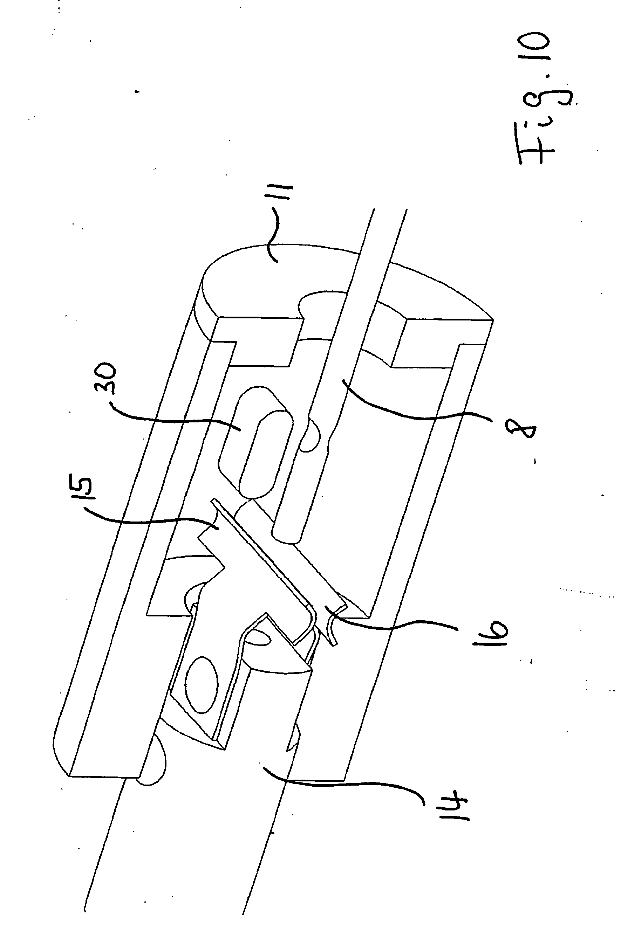

Fig. 10 zeigt eine perspektivische Darstellung mit Nadelanschlusseinrichtung 14 und Betätigungselement 11 nach einer weiteren Ausführungsform. Im Unterschied zu der Ausführungsform in den Fig. 3, 4, 6 und 7 werden die Funktionselemente 15, 16 der Nadelanschlusseinrichtung 14 mittels eines Vorsprungs 30 auseinandergedrückt, welcher seinerseits an dem Betätigungselement 11 angeordnet ist.

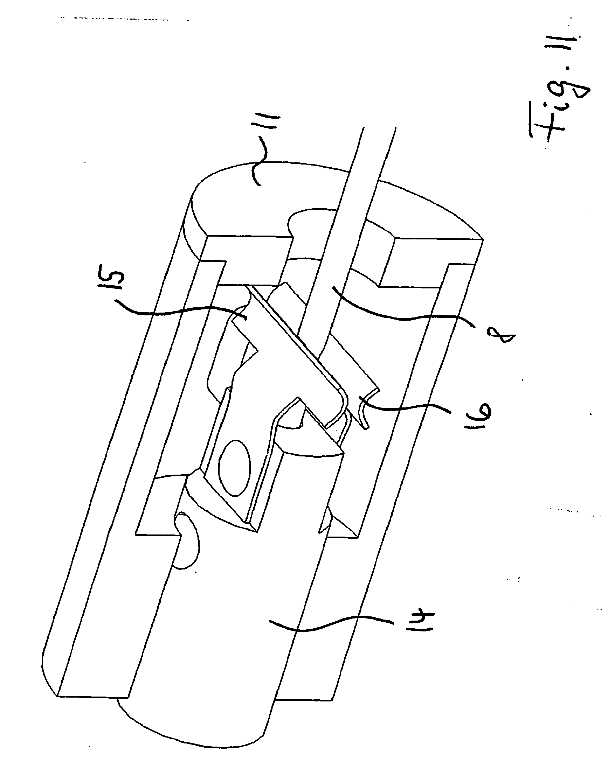

Fig. 11 zeigt eine perspektivische Darstellung der Anordnung aus Fig. 10 im gekoppelten Zustand.

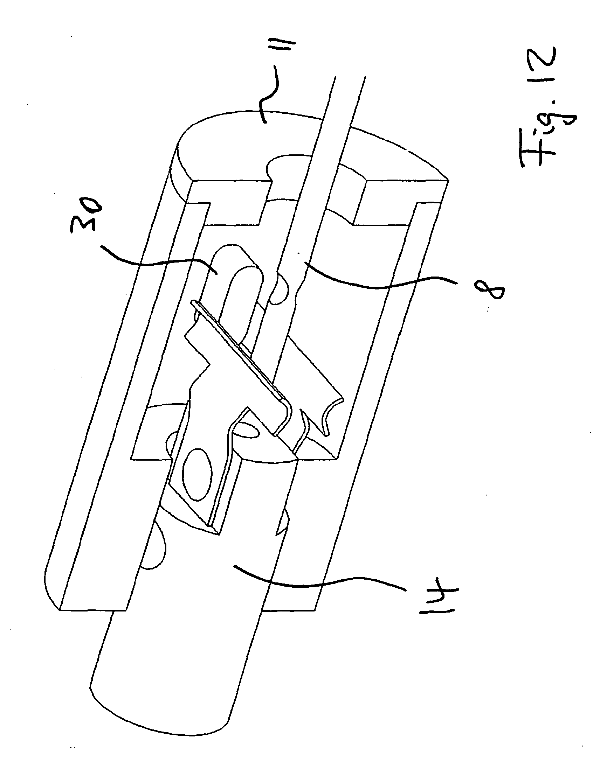

Fig. 12. zeigt eine perspektivische Darstellung der Anordnung aus Fig. 10, wobei die Nadelanschlusseinrichtung 14 in einer Ab- oder Entkopplungsstellung ist. Der Vorsprung 30 drückt hier die Funktionselemente 15, 16 auseinander.

Fig. 13 zeigt eine perspektivische Darstellung eines Abschnitts des Antriebsmoduls 1. Es sind zwei diametral magnetisierte Magnete 40, 41 integriert. Ein oberer Magnet 40 ist in einen Gehäusedeckel 2 eingelassen. Ein unterer Magnet 41 ist in einer Schwungscheibe 43 angeordnet. Die beiden Magneten, 40, 41 sind so ausgerichtet, dass sich bei der in Fig. 13 dargestellten Schwungmassenposition entgegengesetzte Magnetpole gegenüberliegen. Bleibt der Elektromotor des Antriebsmoduls 1 in einer anderen Position stehen, erzeugen die beiden Magnete 40, 41 ein Drehmoment, dass ausreichend ist, um die Schwungmasse im stromlosen Zustand in die in Fig. 13 dargestellte Position zu drehen, wodurch das Nadelelement 6 eingefahren wird. Somit besteht keine Verletzungsgefahr im abgeschalteten Zustand des Antriebsmoduls 1.

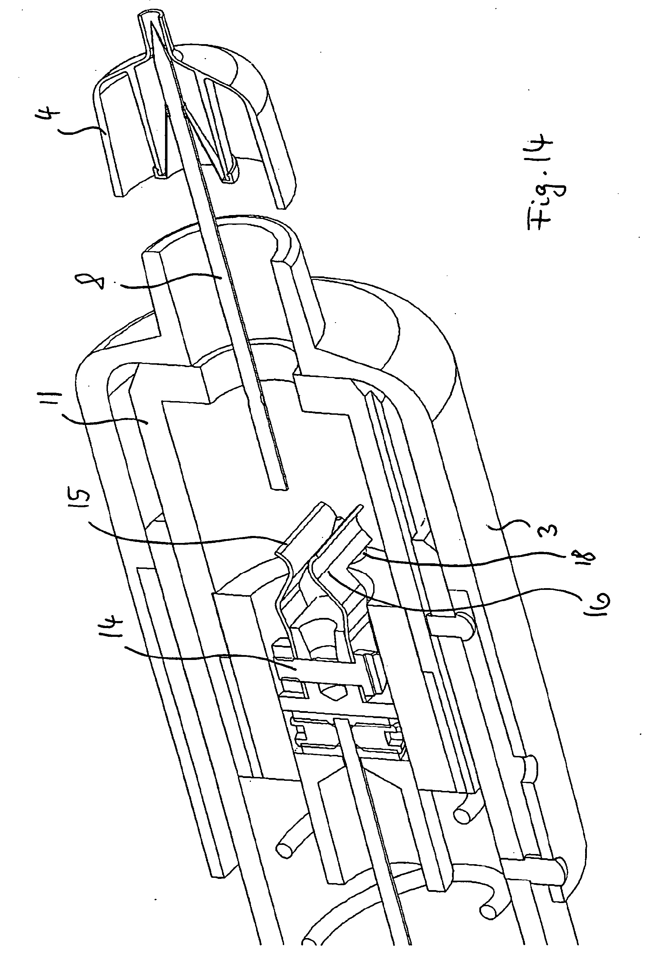

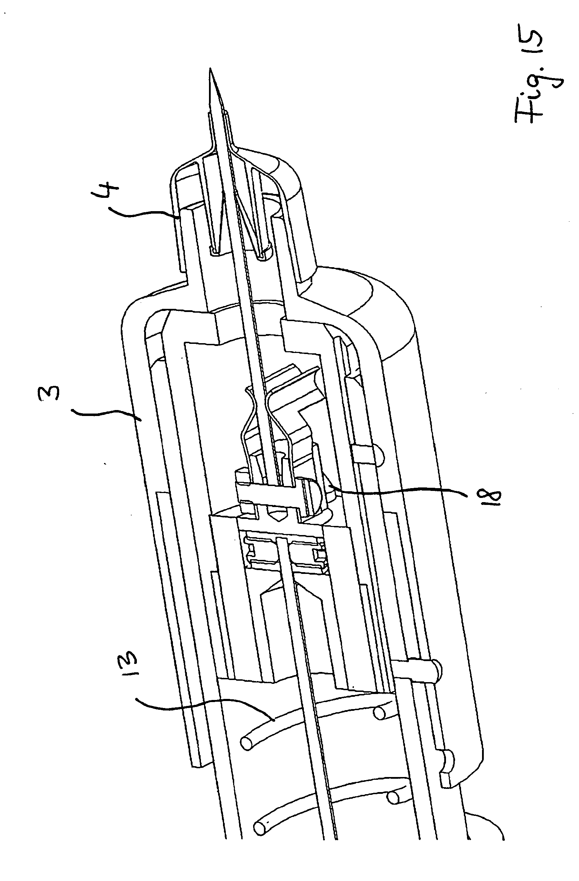

Fig. 14 und 15 zeigen perspektivische Darstellungen eines Handgeräts, bei dem das Nadelmodul 2 mehrteilig ausgeführt ist. Der proximale Abschnitt 3 ist trennbar von dem distalen Abschnitt 4. Beim Ankoppeln des Nadelmoduls 2 an das Antriebsmodul 1 kann zunächst der proximale Abschnitt 4 angekoppelt werden, wodurch das Betätigungselement 11 betätigt wird (vgl. Fig. 14). Anschließend wird der distale Abschnitt 4 mit der Nadeleinrichtung 8 aufgesteckt (vgl. Fig. 15).

Weiterhin kann in der in Fig. 14 dargestellten Ausgestaltung der proximale Abschnitt 3 des Nadelmoduls 2 an dem Antriebsmodul 1 zwei Rastpositionen einnehmen, derart, dass das Betätigungselement 11 des Antriebsmoduls 1 zeitgleich die erste Verlagerungsstellung einnimmt, wenn der proximale Abschnitt 3 die erste Rastposition einnimmt, und dass das zugehörige Betätigungselement 11 des Antriebsmoduls zeitgleich die zweite Verlagerungsstellung einnimmt, wenn der proximale Abschnitt 3 die zweite Rastposition einnimmt. Hierdurch wird die Nadelanschlusseinrichtung 8 in Kopplungs- / Abkopplungsstellung gebracht, wenn der proximale Abschnitt 3 des Nadelmoduls 2 sich in der zweiten bzw. ersten Raststellung befindet. Dadurch kann der distale Abschnitt 4 des Nadelmoduls von dem proximalen Abschnitt 3 abgezogen oder hiermit verbunden werden, wenn der proximale Abschnitt 3 die erste Rastposition einnimmt. Somit kann der distale Abschnitt 4 gewechselt werden, während der proximale Abschnitt 3 des Nadelmoduls 2 mit dem Antriebsmodul 1 verbunden bleibt.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Handgerät zum lokalen Aufstechen einer menschlichen oder tierischen Haut, insbesondere zum Einbringen eines Wirkstoffs, zum Tätowieren oder zum Aufbringen von permanentem Make-up, mit:
- einem Antriebsmodul (1), welches konfiguriert ist, eine repetierende Antriebsbewegung bereitzustellen, und
- einem Nadelmodul (2), welches eine Nadeleinrichtung (8) aufweist und konfiguriert ist, an das Antriebsmodul (1) zu koppeln, derart, dass die repetierende Antriebsbewegung zum Ein- / Ausfahren der Nadeleinrichtung (8) auf diese einkoppelbar ist,
wobei an dem Antriebsmodul (1) ein verlagerbares, an eine Nadelanschlusseinrichtung (14) funktionell gekoppeltes Betätigungselement (11) und an dem Nadelmodul (2) ein dem Betätigungselement (11) zugeordnetes Funktionselement (10) gebildet sind, die konfiguriert sind, beim Ankoppeln des Nadelmoduls (2) an das Antriebsmodul (1) mit dem zugeordneten Funktionselement (10) das Betätigungselement (11) aus einer ersten in eine zweite Verlagerungsstellung zu verlagern, wodurch die Nadelanschlusseinrichtung (14) in eine Kopplungsstellung gebracht wird, in welcher die Nadeleinrichtung (8) zum Betrieb an die Nadelanschlusseinrichtung (14) koppelbar ist.

2. Handgerät nach Anspruch 1, **dadurch gekennzeichnet, dass** das Betätigungselement (11) konfiguriert ist, sich beim Abkoppeln des Nadelmoduls (2) von dem Antriebsmodul (1) selbsttätig aus der zweiten in die erste Verlagerungsstellung zu verlagern, wodurch die Nadelanschlusseinrichtung (14) in eine Abkopplungsstellung gebracht wird, in welcher die Nadeleinrichtung (8) von der Nadelanschlusseinrichtung (14) abkoppelbar ist.

3. Handgerät nach Anspruch 1 oder 2, **dadurch gekennzeichnet, dass** das Antriebsmodul (1) und das Nadelmodul (2) konfiguriert sind, eine formschlüssige Kopplungsverbindung einzugehen.

4. Handgerät nach mindestens einem der vorangehenden Ansprüche, **dadurch gekennzeichnet, dass** das Nadelmodul (2) einen proximalen Abschnitt (3), an dem das zugeordnete Funktionselement (10) gebildet ist und welcher konfiguriert ist, an das Antriebsmodul (1) zu koppeln, und einen hiermit verbundenen distalen Abschnitt (4) aufweist, in welchem eine Nadelöffnung (5) gebildet ist, durch welche hindurch ein Nadelelement (6) der Nadeleinrichtung (8) ein- und ausgefahren werden kann.

5. Handgerät nach Anspruch 4, **dadurch gekennzeichnet, dass** der proximale Abschnitt (3) und der distale Abschnitt (4) lösbar miteinander verbunden sind.

6. Handgerät nach Anspruch 4 oder 5, **dadurch gekennzeichnet, dass** in einer angekoppelten Stellung des Nadelmoduls (2) an dem Antriebsmodul (1) der proximale Abschnitt (3) an dem Antriebsmodul (1) fixiert ist.

7. Handgerät nach mindestens einem der Ansprüche 4 bis 6, **dadurch gekennzeichnet, dass** beim Ankoppeln des Nadelmoduls (2) an das Antriebsmodul (1) der proximale Abschnitt (3) an einem Gehäuse (12) des Antriebsmoduls (1) geführt ist.

8. Handgerät nach mindestens einem der vorangehenden Ansprüche, **dadurch gekennzeichnet, dass** das Betätigungselement (11) konfiguriert ist, sich zwischen der ersten und der zweiten Verlagerungsstellung in Richtung einer beim Ankoppeln des Nadelmoduls (2) an das Antriebsmodul (1) von dem Nadelmodul (2) ausgeführten Ankopplungsbewegung zu verlagern.

9. Handgerät nach mindestens einem der vorangehenden Ansprüche, **dadurch gekennzeichnet, dass** das Betätigungselement (11) in einer Führung an dem Antriebsmodul (1) gelagert ist, welche konfiguriert ist, das Betätigungselement (11) beim Übergang zwischen der ersten und der zweiten Verlagerungsstellung zu führen.

10. Handgerät nach mindestens einem der vorangehenden Ansprüche, **dadurch gekennzeichnet, dass** die Nadelanschlusseinrichtung (14) mit einem mechanischen Anschluss-Mechanismus für die Nadeleinrichtung (8) gebildet ist.

11. Handgerät nach Anspruch 10, **dadurch gekennzeichnet, dass** die Nadelanschlusseinrichtung (14) einander zugeordnete und relativ zueinander verlagerbare Klemm- oder Befestigungselemente (15, 16) aufweist.

12. Handgerät nach mindestens einem der vorangehenden Ansprüche, **dadurch gekennzeichnet, dass** die Nadelanschlusseinrichtung (14) konfiguriert ist, die Nadeleinrichtung (8) starr anzukoppeln.

13. Handgerät nach mindestens einem der vorangehenden Ansprüche, **dadurch gekennzeichnet, dass** die Nadeleinrichtung (8) in dem Nadelmodul (2) in einem die Nadeleinrichtung (8) beim Ankoppeln des Nadelmoduls (2) an das Antriebsmodul (1) in einer Ausgangsstellung haltenden Führungselement (9) gelagert ist.

14. Verfahren zum Ankoppeln eines Nadelmoduls (2) an ein Antriebsmodul (1) bei einem Handgerät nach mindestens einem der Ansprüche 1 bis 13, wobei das Verfahren die folgenden Schritte umfasst:
- Bereitstellen eines Antriebsmoduls (1), welches konfiguriert ist, eine repetierende Antriebsbewegung bereitzustellen,
- Bereitstellen eines Nadelmoduls (2), welches eine Nadeleinrichtung (8) aufweist und konfiguriert ist, an das Antriebsmodul (1) zu koppeln, derart, dass die repetierende Antriebsbewegung zum Ein- / Ausfahren der Nadeleinrichtung (8) auf diese einkoppelbar ist, und
- Ankoppeln des Nadelmoduls (2) an das Antriebsmodul (1), indem ein verlagerbares, an eine Nadelanschlusseinrichtung (14) funktionell gekoppeltes Betätigungselement (11) des Antriebsmoduls (1) mittels eines dem Betätigungselement (11) zugeordneten Funktionselement (10) des Nadelmoduls (2) aus einer ersten in eine zweite Verlagerungsstellung verlagert wird, wodurch die Nadelanschlusseinrichtung (14) in eine Kopplungsstellung gebracht wird, und indem die Nadeleinrichtung (8) dann zum Betrieb an die Nadelanschlusseinrichtung (14) gekoppelt wird.

15. Antriebsmodul (1) für ein Handgerät nach mindestens einem der Ansprüche 1 bis 13, mit:
- Antriebsmitteln, welche konfiguriert sind, eine repetierende Antriebsbewegung bereitzustellen,
- einer Nadelanschlusseinrichtung (14) und
- einem verlagerbaren Betätigungselement (11), welches an die Nadelanschlusseinrichtung (14) funktionell gekoppelt und konfiguriert ist, beim Ankoppeln eines Nadelmoduls (2) mit einem hieran gebildeten und dem Betätigungselement (11) zugeordneten Funktionselement (10) das Betätigungselement (11) aus einer ersten in eine zweite Verlagerungsstellung zu verlagern, wodurch die Nadelanschlusseinrichtung (14) in eine Kopplungsstellung gebracht wird, in welcher eine von dem Nadelmodul (2) umfassten Nadeleinrichtung (8) zum Betrieb an die Nadelanschlusseinrichtung (14) koppelbar ist.

16. Nadelmodul (2) für ein Handgerät nach mindestens einem der Ansprüche 1 bis 13, mit:
- einer Nadeleinrichtung (8) und
- einem Funktionselement (10), welches einem verlagerbaren Betätigungselement (11) eines ankoppelbaren Antriebsmoduls (1) zugeordnet und konfiguriert ist, beim Ankoppeln an das Antriebsmodul (1) das Betätigungselement (11) aus einer ersten in eine zweite Verlagerungsstellung zu verlagern, wodurch eine von dem Antriebsmodul (1) umfasste Nadelanschlusseinrichtung (14) in eine Kopplungsstellung gebracht wird, in welcher die Nadeleinrichtung (8) zum Betrieb an die Nadelanschlusseinrichtung (14) koppelbar ist.

## Claims

1. A handheld device for the local puncturing of a human or an animal skin, in particular for the introduction of an active substance or for the application of a tattoo or a permanent make-up, comprising:
- a drive unit (1) which is configured so as to produce a repetitive drive movement; and
- a needle unit (2) which comprises a needle device (8) and is configured so as to couple to the drive unit (1) in such a manner that the repetitive drive movement for the extension and retraction of the needle device (8) can be coupled into the latter;
wherein said drive unit (1) having formed on it a displaceable actuating element (11) which is functionally coupled to a needle connection device (14), and said needle unit (2) having formed on it a functional member (10) which is assigned to the actuating element (11), said actuating element (11) and said functional member (10) being configured so as to move the actuating element (11) from a first displacement position to a second displacement position by means of the assigned functional member (10) when coupling the needle unit (2) to the drive unit (1), thus moving the needle connection device (14) to a coupling position in which the needle device (8) can be coupled to the needle connection device (14) for purposes of operation.

2. The handheld device according to claim 1, **characterized in that** the actuating element (11) is configured so as to move automatically from the second displacement position to the first displacement position when the needle unit (2) is uncoupled from the drive unit (1), thus moving the needle connection device (14) to an uncoupling position in which the needle device (8) can be uncoupled from the needle connection device (14).

3. The handheld device according to claim 1 or 2, **characterized in that** the drive unit (1) and the needle unit (2) are configured so as to form a positive coupling connection.

4. The handheld device according to at least one of the preceding claims, **characterized in that** the needle unit (2) comprises a proximal section (3) on which is formed the assigned functional member (10) and which is configured so as to couple to the drive unit (1), and a distal section connected thereto in which is formed a needle opening (5) through which a needle element (6) of the needle device (8) can be retracted and extended.

5. The handheld device according to claim 4, **characterized in that** the proximal section (3) and the distal section (4) are detachably connected to each other.

6. The handheld device according to claim 4 or 5, **characterized in that** the proximal section (3) is fixed to the drive unit (1) when the needle unit (2) is coupled to the drive unit (1).

7. The handheld device according to at least one of claims 4 to 6, **characterized in that** the proximal section (3) is guided on a housing (12) of the drive unit (1) during the coupling process of the needle unit (2) to the drive unit (1).

8. The handheld device according to at least one of the preceding claims, **characterized in that** the actuating element (11) is configured so as to move between the first and the second displacement position in the direction of a coupling movement carried out by the needle unit (2) during the coupling process of the needle unit (2) to the drive unit (1).

9. The handheld device according to at least one of the preceding claims, **characterized in that** the actuating element (11) is mounted in a guiding device on the drive unit (1) which is configured so as guide the actuating element (11) when moving from the first to the second displacement position.

10. The handheld device according to at least one of the preceding claims, **characterized in that** the needle connection device (14) is formed with a mechanical connection mechanism for the needle device (8).

11. The handheld device according to claim 10, **characterized in that** the needle connection device (14) has clamping elements or fixation elements (15, 16) which are assigned to each other and displaceable relative to each other.

12. The handheld device according to at least one of the preceding claims, **characterized in that** the needle connection device (14) is configured so as to rigidly couple to the needle device (8).

13. The handheld device according to at least one of the preceding claims, **characterized in that** the needle device (8) in the needle unit (2) is mounted in a guiding element (9) which holds the needle device (8) in an initial position when the needle unit (2) is coupled to the drive unit (1).

14. A method for coupling a needle unit (2) to a drive unit (1) on a handheld device according to at least one of claims 1 to 13 wherein, said method comprising the following steps:
- provision of a drive unit (1) which is configured so as to produce a repetitive drive movement;
- provision of a needle unit (2) which comprises a needle device (8) and is configured so as to couple to the drive unit (1) in such a manner that the repetitive drive movement for the extension and retraction of the needle device (8) can be coupled into the latter; and
- coupling of the needle unit (2) to the drive unit (1) by moving a displaceable actuating element (11) of the drive unit (1), which is functionally coupled to a needle connection device (14), from a first displacement position to a second displacement position by means of a functional member (10) of the needle unit (2) which is assigned to the actuating element (11), thus moving the needle connection device (14) into a coupling position in which the needle device (8) is coupled to the needle connection device (14) for purposes of operation.

15. A drive unit (1) for a handheld device according to at least one of claims 1 to 13, comprising:
- drive means which are configured so as to produce a repetitive drive movement;
- a needle connection device (14); and
- a displaceable actuating element (11) which is functionally coupled to the needle connection device (14) and configured so as to move the actuating element (11) from a first displacement position to a second displacement position when a needle unit (2) having formed on it a functional member (10) which is assigned to the actuating element (11) is coupled to said drive unit (1), thus moving the needle connection device (14) to a coupling position in which a needle device (8) encompassed by the needle unit (2) can be coupled to the needle connection device (14) for purposes of operation.

16. A needle unit (2) for a handheld device according to at least one of claims 1 to 13, comprising:
- a needle device (8), and
- functional member (10) which is assigned to a displaceable actuating element (11) of a drive unit (1) which can be coupled to said needle unit (2) and being configured so as to move the actuating element (11) from a first displacement position to a second displacement position when the needle unit (2) is coupled to the drive unit (1), thus moving a needle connection device (14) encompassed by the drive unit (1) to a coupling position in which the needle device (8) can be coupled to the needle connection device (14) for purposes of operation.

## Revendications

1. Appareil à main pour le perçage local d'une peau humaine ou d'une peau animale, en particulier pour l'introduction d'une matière active, pour le tatouage ou pour l'application de fond de teint permanent, avec :
u n module d'entraînement (1) qui est configuré de manière à réaliser un mouvement d'entraînement répétitif, et
u n module à aiguille (2) qui présente un équipement à aiguille (8) et est configuré de manière à se coupler au module d'entraînement (1) de telle sorte que le mouvement d'entraînement répétitif puisse être couplé à l'équipement à aiguille (8) pour l'entrée et la sortie de ce dernier,
étant donné que sont formés sur le module d'entraînement (1) un élément d'actionnement (11) décalable couplé fonctionnellement à un équipement de raccord d'aiguille (14) et sur le module à aiguille (2) un élément fonctionnel (10) affecté à l'élément d'actionnement (11), élément configurés de manière à décaler, à l'accouplement du module à aiguille (2) avec le module d'entraînement (1), l'élément d'actionnement (11) avec l'élément fonctionnel (10) correspondant d'une première position de décalage à une deuxième position de décalage, ce par quoi l'équipement de raccord d'aiguille (14) est amené à une position de couplage à laquelle l'équipement à aiguille (8) peut être couplé à l'équipement de raccord d'aiguille (14) pour fonctionner.

2. Appareil à main selon la revendication 1, **caractérisé en ce que** l'élément d'actionnement (11) est configuré de manière à se déplacer de façon autonome de la deuxième à la première position de décalage au désaccouplement du module à aiguille (2) du module d'entraînement (1), ce par quoi l'équipement de raccord d'aiguille (14) est amené à une position de désaccouplement à laquelle l'équipement à aiguille (8) peut être désaccouplé de l'équipement de raccord d'aiguille (14).

3. Appareil à main selon la revendication 1 ou 2, **caractérisé en ce que** le module d'entraînement (1) et le module à aiguille (2) sont configurés de manière à être en liaison de couplage crabotée.

4. Appareil à main selon au moins l'une quelconque des revendications précédentes, **caractérisé en ce que** le module à aiguille (2) présente une section proximale (3) à laquelle est formé l'élément fonctionnel (10) correspondant et qui est configurée de manière à accoupler le module d'entraînement (1), et une section distale (4) reliée à ce dernier, section distale dans laquelle est formée un orifice à aiguille (5) à travers lequel un élément d'aiguille (6) de l'équipement à aiguille (8) peut être introduit et sorti.

5. Appareil à main selon la revendication 4, **caractérisé en ce que** la section proximale (3) et la section distale (4) sont reliées de manière amovible l'une avec l'autre.

6. Appareil à main selon la revendication 4 ou 5, **caractérisé en ce que**, à une position à laquelle le module à aiguille (2) est accouplé avec le module d'entraînement (1), la section proximale (3) est fixée au module d'entraînement (1).

7. Appareil à main selon au moins l'une quelconque des revendications 4 ou 6, **caractérisé en ce que**, à l'accouplement du module à aiguille (2) avec le module d'entraînement (1), la section proximale (3) est guidée à un boîtier (12) du module d'entraînement (1).

8. Appareil à main selon au moins l'une quelconque des revendications précédentes, **caractérisé en ce que** l'élément d'actionnement (11) est configuré de manière à se déplacer entre la première et la deuxième position de décalage dans le sens d'un mouvement d'accouplement accompli par le module à aiguille (2) à l'accouplement du module à aiguille (2) avec le module d'entraînement (1).

9. Appareil à main selon au moins l'une quelconque des revendications précédentes, **caractérisé en ce que** l'élément d'actionnement (11) est logé dans une coulisse sur le module d'entraînement (1) configurée de manière à guider l'élément d'actionnement (11) à la transition de la première position de décalage à la deuxième position de décalage.

10. Appareil à main selon au moins l'une quelconque des revendications précédentes, **caractérisé en ce que** l'équipement de raccord d'aiguille (14) est formé avec un mécanisme de raccordement mécanique pour l'équipement à aiguille (8).

11. Appareil à main selon la revendication 10, **caractérisé en ce que** l'équipement de raccord d'aiguille (14) présente des éléments de serrage et de fixation (15, 16) affectés l'un à l'autre et décalables l'un par rapport à l'autre.

12. Appareil à main selon au moins l'une quelconque des revendications précédentes, **caractérisé en ce que** l'équipement de raccord d'aiguille (14) est configuré de manière à s'accoupler rigidement à l'équipement à aiguille (8).

13. Appareil à main selon au moins l'une quelconque des revendications précédentes, **caractérisé en ce que** l'équipement à aiguille (8) est logé dans le module à aiguille (2) dans un élément de guidage (9) qui maintient l'équipement à aiguille (8) à une position de départ à l'accouplement du module à aiguille (2) avec le module d'entraînement (1).

14. Procédé pour l'accouplement d'un module à aiguille (2) avec un module d'entraînement (1) sur un appareil à main selon au moins l'une quelconque des revendications 1 à 13, le procédé comprenant les étapes suivantes :
- mise à disposition d'un module d'entraînement (1) qui est configuré de manière à réaliser un mouvement d'entraînement répétitif,
- mise à disposition d'un module à aiguille (2) qui présente un équipement à aiguille (8) et est configuré de manière à se coupler au module d'entraînement (1) de telle sorte que le mouvement d'entraînement répétitif puisse être couplé à l'équipement à aiguille (8) pour l'entrée et la sortie de ce dernier, et
- accouplement du module à aiguille (2) au module d'entraînement (1) par décalage d'un élément d'actionnement (11) décalable et fonctionnellement couplé à un équipement de raccord d'aiguille (14) du module d'entraînement (1) au moyen d'un élément fonctionnel (10) du module à aiguille (2), affecté à l'élément d'actionnement (11), d'une première position de décalage à une deuxième position de décalage, ce par quoi l'équipement de raccord d'aiguille (14) est amené à une position de couplage, puis par couplage de l'équipement à aiguille (8) pour le fonctionnement à l'équipement de raccord d'aiguille (14).

15. Module d'entraînement (1) pour un appareil à main selon au moins l'une quelconque des revendications 1 à 13, avec :
- des moyens d'entraînement configurés de manière à réaliser un mouvement d'entraînement répétitif,
- un équipement de raccord d'aiguille (14) et
- un élément d'actionnement (11) décalable couplé fonctionnellement à l'équipement de raccord d'aiguille (14) et configuré de manière à décaler, à l'accouplement d'un module à aiguille (2) avec un élément fonctionnel (10) formé sur ce dernier et affecté à l'élément d'actionnement (11), ledit élément d'actionnement (11) d'une première position de décalage à une deuxième position de décalage, ce par quoi l'équipement de raccord d'aiguille (14) est amené à une position de couplage à laquelle un équipement à aiguille (8) que comprend le module à aiguille (2) peut être couplé à l'équipement de raccord d'aiguille (14) pour fonctionner.

16. Module à aiguille (2) pour un appareil à main selon au moins l'une quelconque des revendications 1 à 13, avec :
- un équipement à aiguille (8) et
- un élément fonctionnel (10) qui est affecté à un élément d'actionnement (11) décalable d'un module d'entraînement (1) accouplable et est configuré de manière à ce que, à l'accouplement avec le module d'entraînement (1), l'élément d'actionnement (11) soit décalé d'une première position de décalage à une deuxième position de décalage, ce par quoi un équipement de raccord d'aiguille (14) que comprend le module d'entraînement (1) est amené à une position à laquelle l'équipement à aiguille (8) peut être couplé à l'équipement de raccord d'aiguille (14) pour fonctionner.
